# EUROPÄISCHE PATENTSCHRIFT

(11) **EP 0 731 084 B1**
(45) Veröffentlichungstag und Bekanntmachung des Hinweises auf die Patenterteilung: **25.09.2002**
(21) Anmeldenummer: 96102561.6
(22) Anmeldetag: 21.02.1996
(51) Int. Cl.: C07C 69/92, C09K 19/32, C07D 303/12, C09K 19/34

(54) **Photovernetzbare Naphthylderivate**
Photocrosslinkable naphthyl derivatives
Dérivés naphtyl photoréticulables

(30) Priorität: 03.03.1995 CH 60795
(43) Veröffentlichungstag der Anmeldung: 11.09.1996
(73) Patentinhaber: Rolic AG, 6301 Zug (CH)
(72) Erfinder: Kelly, Stephen, Beverley HU17 8XA, East Yorkshire (GB)
(74) Vertreter: Liebetanz, Michael

(56) Entgegenhaltungen:
- EP-A- 0 449 049
- DE-A- 19 517 762
- US-A- 5 182 394
- US-A- 5 237 076
- MELISSARIS A P ET AL: "High Modulus and High Tg Thermally Stable Polymers from Di-p-ethynylbenzoyl Ester Monomers: Synthesis, Solid State Polymerization;Processing, and Thermal Properties" MACROMOLECULES (MAMOBX,00249297);94; VOL.27 (10); PP.2675-84, CASE WESTERN RESERVE UNIVERSITY;DEPARTMENT OF MACROMOLECULAR SCIENCE; CLEVELAND; 44106; OH; USA (US), XP002040748

## Beschreibung

Die vorliegende Erfindung betrifft photovernetzbare Naphthyl-Derivate, Gemische, die solche Verbindungen enthalten, sowie ihre Verwendung in vernetztem Zustand für optische Bauelemente.

Flüssigkristalle mit mindestens zwei photochemisch oligomerisierbaren oder polymerisierbaren Gruppen können auf einem Substrat oder in einer Zelle orientiert werden, beispielsweise durch Orientierungsschichten oder in einem Feld. Durch Bestrahlen mit Licht einer geeigneten Wellenlänge werden diese orientierten Flüssigkristalle, versehen mit einer geeigneten Menge eines Photoinitiators, polymerisiert. Die dadurch erzeugte, vernetzte Struktur bleibt auch bei hohen Temperaturen erhalten. Solche Schichten können beispielsweise Teile von Hybridschichten sein, wie sie in den Schweizerischen Patentanmeldungen CH 2016/94 und CH 2017/94 beschrieben sind. So lassen sich optische Bauelemente, wie zum Beispiel Retarder, Wellenleiter, optische Gitter und Filter, integrierte Farbfilter, oder Zellen mit piezoelektrischen und solche mit nicht-linearen optischen (NLO) Eigenschaften, usw., herstellen. Solche optische Bauelemente finden zum Beispiel in Projektionssystemen Einsatz.

Weitere Eigenschaften, wie beispielsweise die Doppelbrechung, der Brechungsindex, die Transparenz, usw. müssen je nach Anwendungsgebiet unterschiedlichen Anforderungen genügen. Beispielsweise sollten Materialien für optische Retarder eine hohe Doppelbrechung aufweisen, damit die Schichtdicke der integrierten optischen Bauelemente auf einem Minimum gehalten werden kann.

Neben dem generellen Interesse an photovernetzbaren Flüssigkristallen für optische Bauelemente, eignen sich solche flüssigkristalline Materialien als Cladding von Glasfibern für optische Datenübertragung. Der Einsatz solcher Materialien erhöht den elastischen Modulus in der Längsachse der Fiber, verkleinert die thermischen Expansionskoeffizienten und vermindert Mikroverbiegungsverluste. Dies führt zu einer erhöhten mechanischen Stabilität.

Die photovernetzbaren Flüssigkristalle müssen eine gute chemische und thermische Stabilität, gute Löslichkeit in gängigen Lösungsmitteln und eine gute Stabilität gegenüber elektrischen Feldern und elektromagnetischer Strahlung besitzen. Weiterhin sollten sie von etwa 25°C bis etwa 80°C, wenn möglich von etwa 25°C bis etwa 100°C, eine geeignete Mesophase besitzen, beispielsweise für die oben genannten Anwendungen eine breite smektische oder nematische Mesophase bzw. chiral smektische oder cholesterische Mesophase.

Da Flüssigkristalle in der Regel als Mischungen mehrerer Komponenten zur Anwendung gelangen, ist es wichtig, dass die Komponenten untereinander gut mischbar sind. Herkömmliche photochemisch oligomerisierbare oder polymerisierbare Flüssigkristalle besitzen in der Regel einen hohen Schmelz- und Klärpunkt. Dies hat den Nachteil, dass vorzeitig eine spontane, thermische Polymerisation eintreten kann bei der Verarbeitung, welche bei Temperaturen knapp unter dem Klärpunkt durchgeführt wird, weil bei dieser Temperatur die Viskosität im flüssigkristallinen Zustand am niedrigsten und daher günstig für eine gute Orientierbarkeit ist. Diese spontane Polymerisation führt zu Domänenbildung, wodurch die optischen und thermischen Eigenschaften in den erzeugten, vernetzten Schichten deutlich beeinträchtigt werden. Der Schmelzpunkt kann durch die Herstellung komplizierter Mischungen mit mehreren Komponenten herabgesetzt werden, was zwar eine Verarbeitung bei niedrigeren Temperaturen erlaubt, aber die Gefahr einer Kristallisation der herkömmlichen polymerisierbaren Flüssigkristalle mit sich bringt. Photochemisch oligomerisierbare oder polymerisierbare Verbindungen werden beispielsweise in EP-A-0 331 233 beschrieben.

Es stellte sich somit die Aufgabe, insbesondere für die Anwendung in optischen Filtern, photochemisch oligomerisierbare oder polymerisierbare Verbindungen herzustellen, welche sich durch eine besonders hohe optische Anisotropie Δn auszeichnen und gleichzeitig niedrigere Schmelzund Klärpunkte besitzen, damit sie bei Temperaturen oberhalb Raumtemperatur im flüssigkristallinen Zustand und auch in Lösung sehr gut verarbeitbar sind. Weiter sollten sie möglichst domänenfrei orientierbar und strukturierbar sein und auch eine ausgezeichnete thermische Stabilität und Langzeitstabilität im vernetzten Zustand aufweisen.

Die vorliegende Erfindung stellt nun Verbindungen zur Verfügung, die in hervorragender Weise als Einzelkomponenten oder als Komponenten von Flüssigkristall-Mischungen für solche optischen Bauelemente geeignet sind, nämlich Verbindungen der allgemeinen Formel 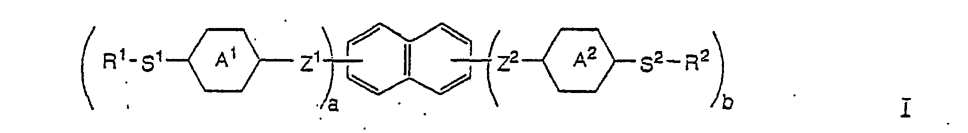 worin
- R¹ und R²: unabhängig voneinander eine vernetzbare Gruppe wie Acrylat, Methacrylat, 2-Chloracrylat, 2-Phenylacrylat, Epoxy oder Vinyloxy
- S¹ und S²: -(CY₂)ₘ-, -O(CY₂)ₘ-, -(CY₂)ₘO-, -(CY₂)ₘCOO-,
-(CY₂)ₘOOC-, -(Si[(CH₃)₂]O)ₘ-,
-OCH₂(Si[(CH₃)₂]O)ₘSi[(CH₃)₂]CH₂O-, oder
-NHCH₂(Si[(CH₃)₂]O)ₘSi[(CH₃)₂]CH₂NH-;
- Y: Wasserstoff, Fluor oder Methyl;
- a, b: 1;
- m: eine ganze Zahl von 1 bis 16;
- A¹ und A²: unabhängig voneinander gegebenenfalls mit Halogen, Cyano, Methyl, Methoxy und/oder Acetyl einfach oder mehrfach substituiertes 1,4-Phenylen, Pyridin-2, 5-diyl, Pyrimidin-2, 5-diyl, trans-1,4-Cyclohexylen oder trans-1,3-Dioxan-2, 5-diyl; und
- Z¹ und Z²: unabhängig voneinander eine Einfachbindung, -CH₂CH₂-, -OCH₂-, -CH₂O-, -COO-, -OOC-, -(CH₂)₄-, -O(CH₂)₃- oder -(CH₂)₃O- bedeuten, wobei der Naphthylring in Stellung 1 und 5, bzw. 1 und 4 mit Z¹ und Z² verknüpft ist.

Die Verbindungen der allgemeinen Formel I zeichnen sich durch ihre verhältnismässig niedrige Viskosität aus. Sie können daher problemlos auf eine geeignete Oberfläche aufgetragen werden. Dies geschieht in der Regel durch Spin-Coating. Da die erfindungsgemässen Verbindungen zudem eine flüssigkristalline Phase aufweisen, können sie vor dem Vernetzen durch Anlegen eines elektrischen Feldes ausgerichtet werden.

Der Ausdruck "gegebenenfalls mit Halogen, Cyano, Methyl, Methoxy und/oder Acetyl einfach oder mehrfach substituiertes 1,4-Phenylen" umfasst im Rahmen der vorliegenden Erfindung 1,4-Phenylen, mit Fluor, Brom, Chlor, Cyano, Methyl, Methoxy oder Acetyl substituiertes 1,4-Phenylen, wie beispielsweise 2- bzw. 3-Fluor-1,4-phenylen, 2,3-, 2,6- bzw. 3,5-Difluor-1,4-phenylen, 2- bzw. 3-Chlor-1,4-phenylen, 2,3-, 2,6- bzw. 3,5-Dichlor-1,4-phenylen, 2- bzw. 3-Brom-1,4-phenylen, 2- bzw. 3-Cyano-1,4-phenylen, 2,3-Dicyano-1,4-phenylen, 2- bzw. 3-Acetyl-1,4-phenylen, 2- bzw. 3-Methyl-1,4-phenylen, 2- bzw. 3-Methoxy-1,4-phenylen,und dergleichen. Besonders bevorzugte Verbindungen der Formel I sind diejenigen, worin die Ringe A¹ und A² gleich sind und 1,4-Phenylen oder 2- bzw. 3-Fluor-1,4-phenylen bedeuten.

Die Spacergruppen S¹ und S² können gegebenenfalls auch chiral sein. Bevorzugte Verbindungen der Formel I sind diejenigen, worin S¹ und S² die gleiche Bedeutung haben. Bevorzugte Spacergruppen sind diejenigen, worin Y Wasserstoff bedeutet und m eine ganze Zahl von 4 bis 12 ist.

2-Phenylacrylat, R¹ und R² sind Acrylat, Methacrylat, 2-Chloracrylat, Epoxy oder Vinyloxy, Reste, welche nach Beschichten des geeigneten Trägers mit Verbindungen der Formel I photochemisch vernetzt werden können. Insbesondere sind diejenigen Verbindungen der Formel I bevorzugt, worin die Reste R¹ und R² die gleiche Bedeutung haben.

Besonders bevorzugte Verbindungen der Formel I sind diejenigen, worin Z¹ und Z² gleich sind und -OCH₂-, -CH₂O-, -COO- oder -OOC- bedeuten.

Verbindungen, welche ganz besonders bevorzugt werden, sind diejenigen der Formeln 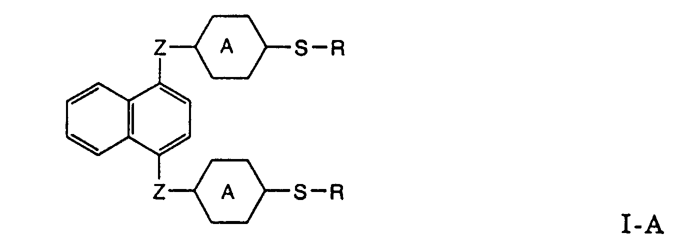 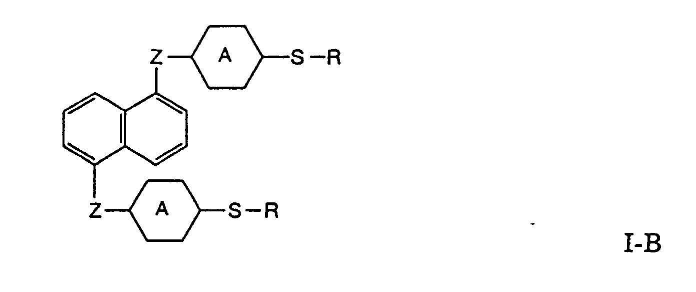 worin
- R: Acrylat, Methacrylat, 2-Chloracrylat, 2-Phenylacrylat, Vinyloxy oder Epoxy;
- S: -(CH₂)_{m'}-, -O(CH₂)_{m'}- oder -(CH₂)_{m'}O- bedeuten;
- m': eine ganze Zahl von 4 bis 12 ist;
- A: 1,4-Phenylen oder 2- bzw. 3-Fluor-1,4-phenylen; und
- Z: -OCH₂- oder -OOC- bedeuten.

Die Verbindungen der Formel I sind synthetisch sehr einfach zugänglich. So können beispielsweise Verbindungen, worin Z eine Gruppe -OOCbedeutet, aus 4-[ω-Acryloxyalkyloxy]benzoesäuren und dem entsprechenden Naphthochinon hergestellt werden (siehe Schemata 1 und 2). Die Veresterung kann in an sich bekannter Weise erfolgen. Eine bevorzugte Methode ist die Umsetzung des Naphthohydrochinon s mit der Carbonsäure in einem polaren, aber inerten organischen Lösungsmittel (z.B. in Dimethylformamid (DMF) oder einem halogeniertem Kohlenwasserstoff, wie Dichlormethan) in Gegenwart von 4-(Dimethylamino)pyridin (DMAP) und N,N'-Dicyclohexylcarbodimid (DCC). Die 4-[ω-Acryloxyalkyloxy]benzoesäuren sind an sich bekannt und können durch Alkylieren von 4-Hydroxybenzoesäure in Gegenwart einer starken Base und eines ω-halogenierten Alkohols sowie Kaliumiodid und anschliessender Veresterung der 4-[ω-Hydroxyalkyloxy]benzoesäuren mit Acrylsäure in Gegenwart von p-Toluolsulfonsäure (PTS) hergestellt werden.

Die in den Schemata verwendeten Symbole haben die obengenannten Bedeutungen.

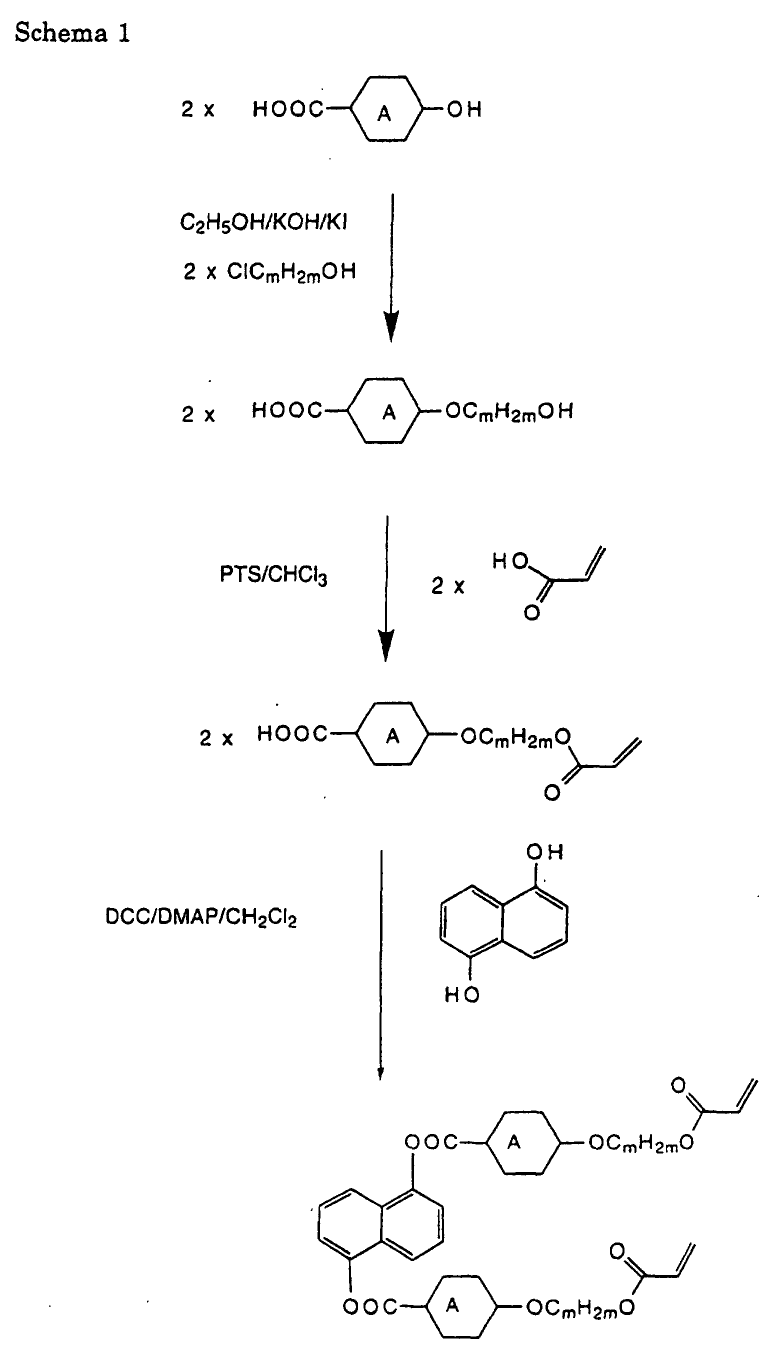

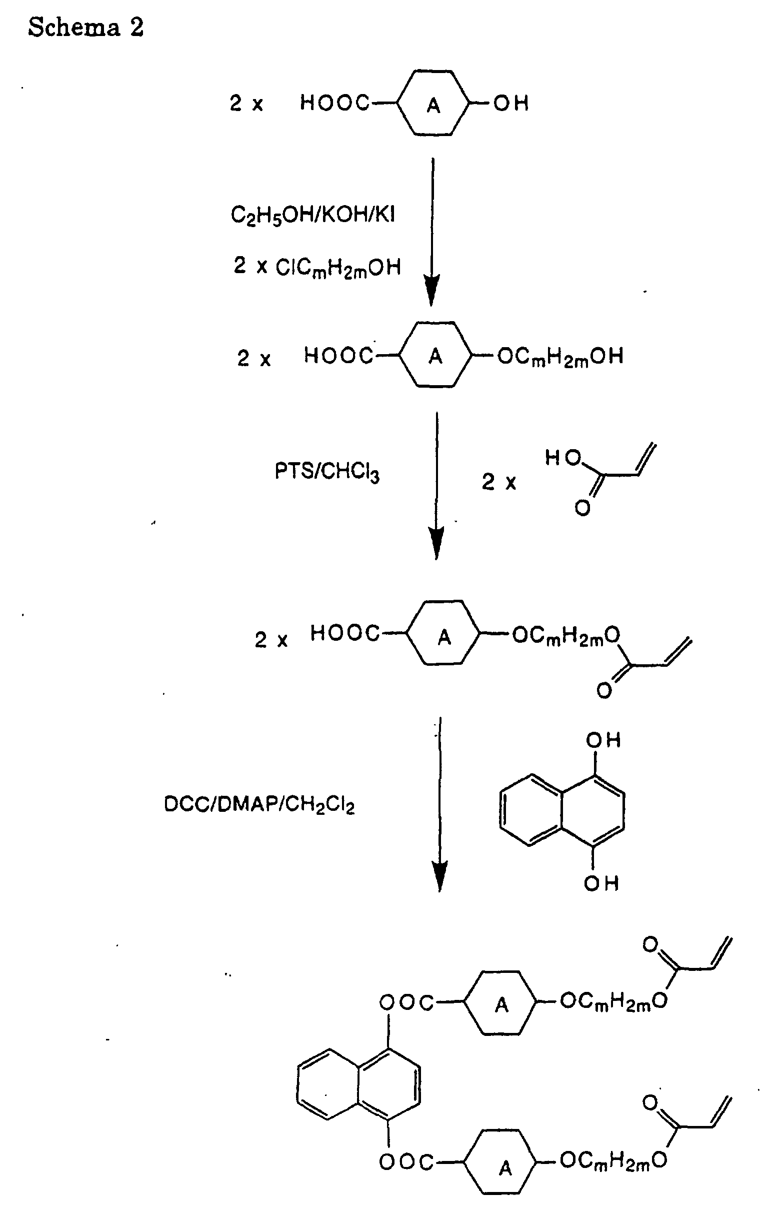

Eine kleine Menge BHT (2, 6-Di-tert.-butyl-4-methyl-phenol/"Butylhydroxytoluol") wird jeder Stufe beigemischt, um unerwünschtes thermisches Vernetzen zu vermeiden.

1,4-bis(4-[ω-Acryloyloxyalkyloxy]phenylmethylenoxy)naphthalin-Derivate, dh. Verbindungen der Formel I, worin Z die Gruppe -OCH₂- bedeutet, können in an sich bekannter Weise durch Verätherung von des entsprechenden Naphthohydrochinon s mit 4-[ω-Acryloyloxyalkyloxy)]benzylalkoholen hergestellt werden. Die Umsetzung kann beispielsweise in Gegenwart von Azodicarbonsäure-dialkylester und Triphenylphosphin erfolgen. Die als Edukt verwendete 4-[ω-Acryloyloxyalkyloxy)]benzylalkohole können durch Reduktion des Reaktionsprodukts von Acrylsäure mit 4-[ω-Hydroxyalkyloxy)]benzylaldehyden hergestellt werden. Die 4-[ω-Hydroxyalkyloxy)]benzylaldehyde können durch Verätherung von 4-Hydroxybenzaldehyd mit ω-Hydroxyalkylhalogeniden in Gegenwart üblicher Basen hergestellt werden.

Die Verbindungen der Formel I können als reine Verbindungen, oder in Form von Gemischen untereinander und/oder mit anderen Flüssigkristallkomponenten verwendet werden.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder auch andere, bekannte flüssigkristalline Verbindungen mit oder ohne photovernetzbare Gruppen sein. Es können auch eine oder mehrere chirale Komponenten im Gemisch enthalten sein.

Aufgrund der guten Löslichkeit der Verbindungen der Formel I und aufgrund ihrer guten Mischbarkeit untereinander kann der Anteil an verschiedenen Verbindungen der Formel I in den erfindungsgemässen Gemischen hoch sein und bis 100 Gew.-% betragen.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 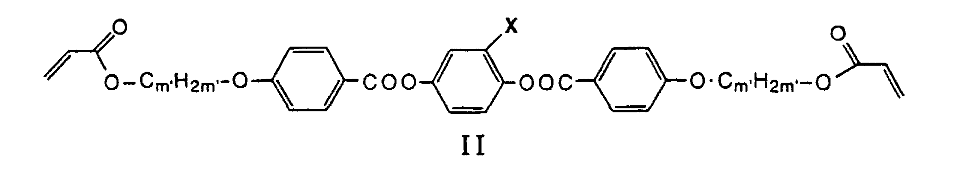 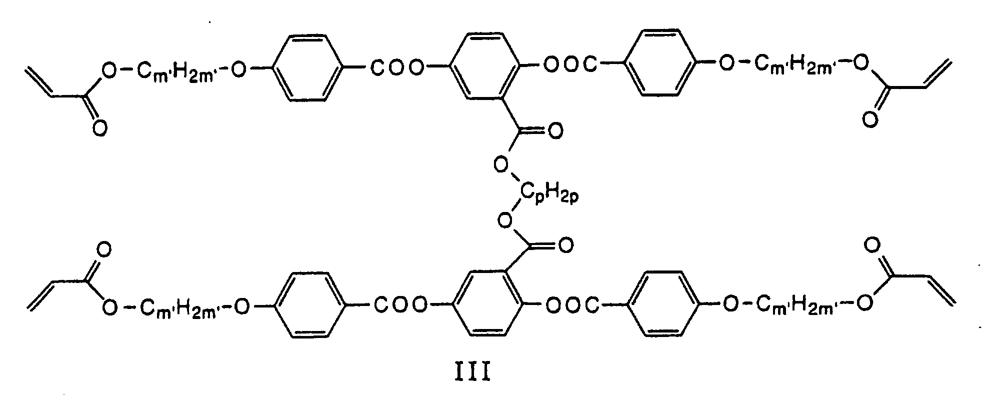 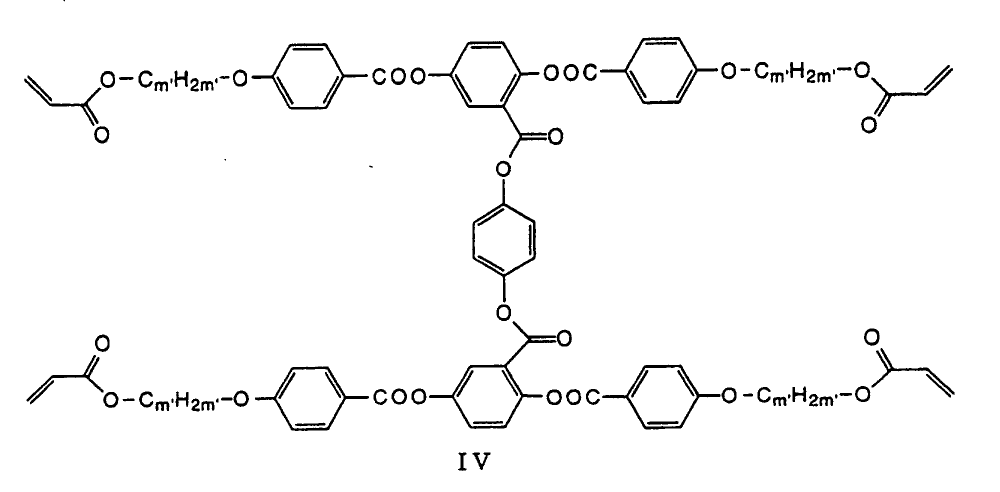 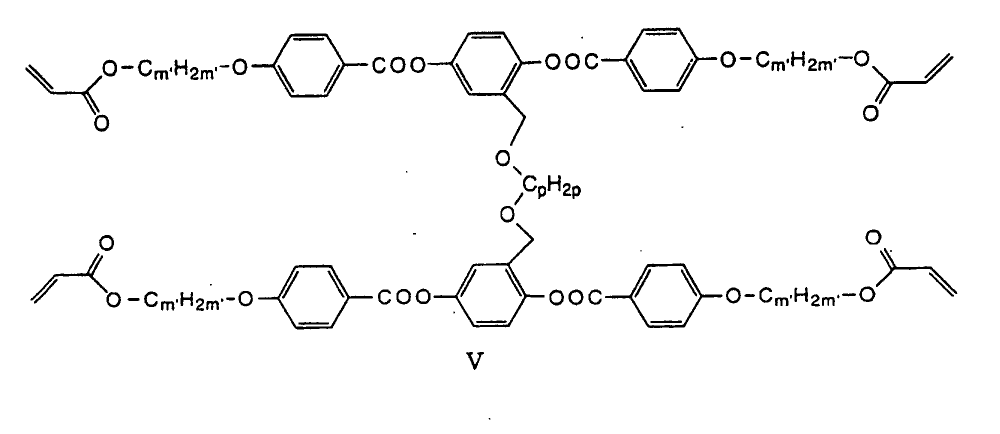 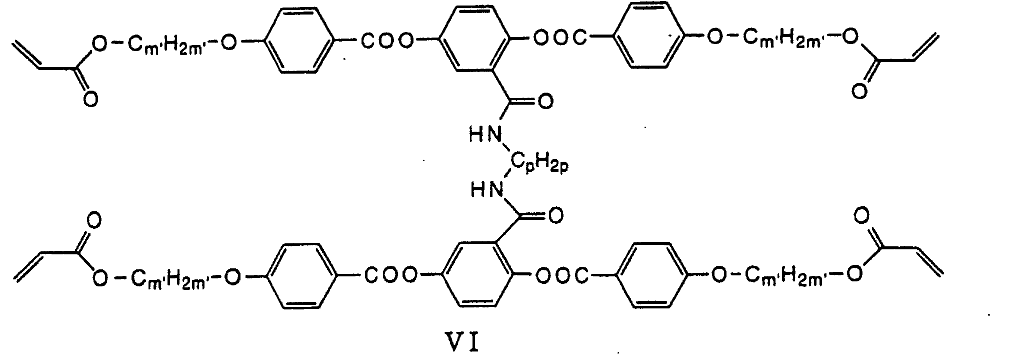 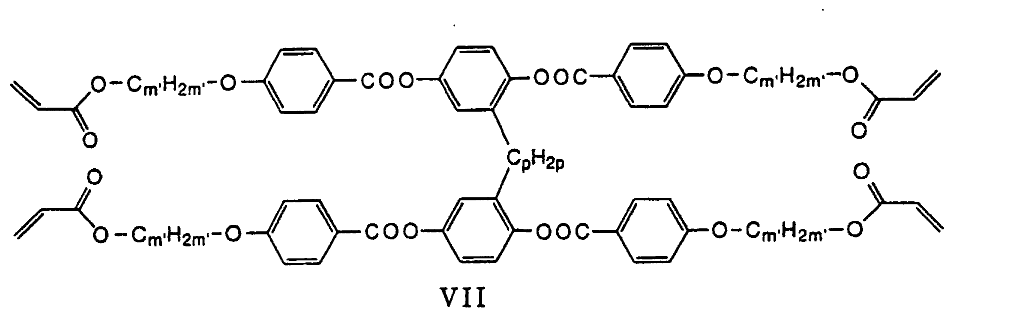 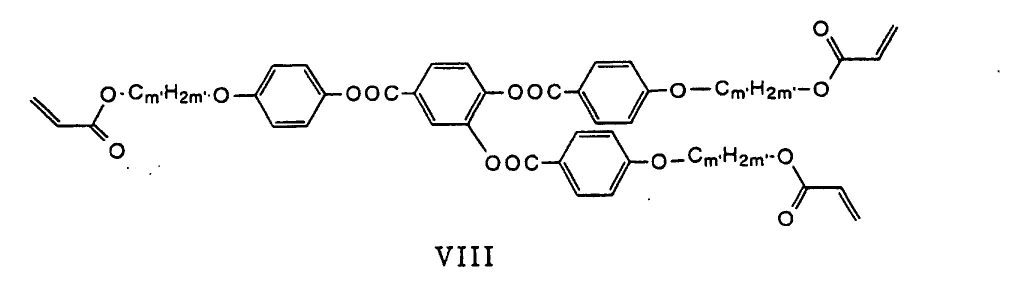 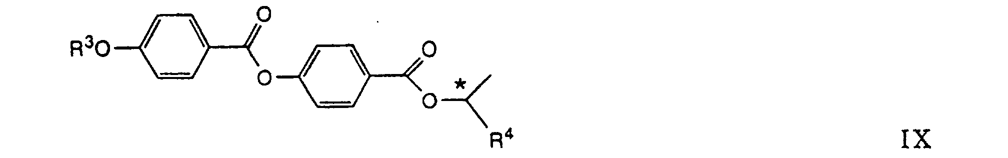 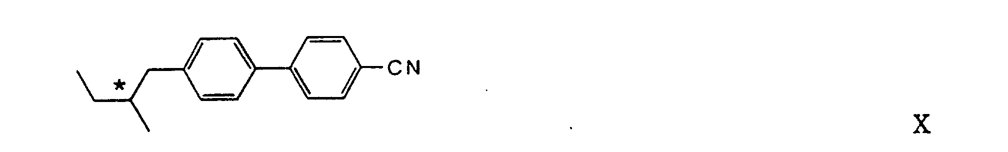 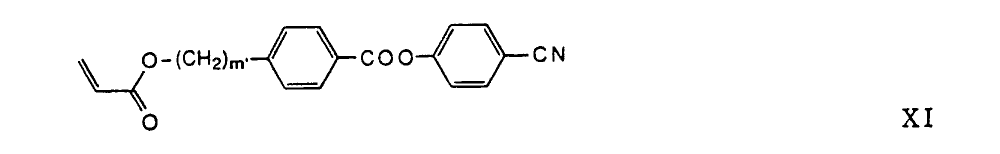 und 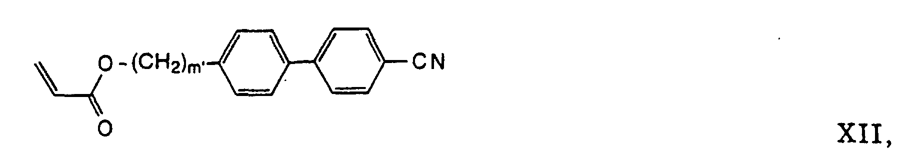 worin
- p: eine ganze Zahl von 2 bis 12;
- R³ und R⁴: unabhängig voneinander Alkyl oder Alkenyl mit 2 bis 12 Kohlenstoffatomen;
- X: Wasserstoff, Niederalkyl, Fluor, Brom, Chlor oder Cyano;
- m': eine ganze Zahl von 4 bis 12; und
- *: ein Chiralitätszentrum bedeuten.

"Niederalkyl" umfasst im Zusammenhang mit der Verbindung der Formel II Methyl, Ethyl, Propyl, i-Propyl, Butyl, i-Butyl und tert.-Butyl.

Die Herstellung der Verbindungen der Formel I sowie flüssigkristalliner Gemische enthaltend diese Verbindungen werden durch die folgenden Beispiele weiter veranschaulicht. C bedeutet eine kristalline, S eine smektische, N eine nematische und I die isotrope Phase.

### Beispiel 1

Zu einer Lösung von 1,25 g 4-[8-Acryloyloxyoctyloxy)]benzoesäure und 0,25 g 1,4-Naphthohydrochinon , 0,2 g 4-Dimethylaminopyridin (DMAP) in 20 ml Dichlormethan wurde unter Rühren bei Raumtemperatur 0,8 g N,N'-Dicyclohexyldicarbodiimid (DCC) gegeben. Das Reaktionsgemisch wurde über Nacht bei Raumtemperatur gerührt, auf 100 ml Wasser gegossen und dann dreimal mit je 50 ml Dichlormethan extrahiert. Die organischen Phasen wurden vereinigt, zweimal mit je 100 ml Wasser gewaschen, über Magnesiumsulfat getrocknet , filtriert und das Filtrat anschliessend eingeengt. Chromatographische Reinigung des Rückstandes an Kieselgel mit Cyclohexan/ Ethylacetat (Vol. 8:2) und zweimaliges Umkristallisieren der gemäss Dünnschichtchromatographie reinen Fraktionen aus Ethylalkohol ergab 0,2 g 1,4-bis(4-[8-Acryloyloxyoctyloxylphenylcarbonyloxy)-naphthalin; Smp. (C-N) 94°C, Klp. (N-I) 104°C.

In analoger Weise können folgende Verbindungen hergestellt werden:
1,4-bis(4-[3-Acryloyloxypropyloxy]phenylcarbonyloxy)naphthalin;
1,4-bis(4-[4-Acryloyloxybutyloxy]phenylcarbonyloxy)naphthalin;
1,4-bis(4-[5-Acryloyloxypentyloxy]phenylcarbonyloxy)naphthalin;
1,4-bis(4-[6-Acryloyloxyhexyloxy]phenylcarbonyloxy)naphthalin;
1,4-bis(4-[7-Acryloyloxyheptyloxy]phenylcarbonyloxy)naphthalin;
1,4-bis(4-[9-Acryloyloxynonyloxy]phenylcarbonyloxy)naphthalin;
1,4-bis(4-[10-Acryloyloxydecyloxy]phenylcarbonyloxy)naphthalin;
1,4-bis(4-[11-Acryloyloxyundecyloxy]phenylcarbonyloxy)naphthalin; Smp. (C-I) 106°C, Klp. (N-I) 98°C.
1,4-bis(4-[12-Acryloyloxydodecyloxy]phenylcarbonyloxy)naphthalin;
1,5-bis(4-[3-Acryloyloxypropyloxy]phenylcarbonyloxy)naphthalin;
1,5-bis(4-[4-Acryloyloxybutyloxy]phenylcarbonyloxy)naphthalin;
1,5-bis(4-[5-Acryloyloxypentyloxy]phenylcarbonyloxy)naphthalin;
1,5-bis(4-[6-Acryloyloxyhexyloxy]phenylcarbonyloxy)naphthalin;
1,5-bis(4-[7-Acryloyloxyheptyloxy]phenylcarbonyloxy)naphthalin;
1,5-bis(4-[8-Acryloyloxyoctyloxy]phenylcarbonyloxy)naphthalin; Smp. (C-I) 130°C, Klp. (N-I) 108°C.
1,5-bis(4-[9-Acryloyloxynonyloxy]phenylcarbonyloxy)naphthalin;
1,5-bis(4-[10-Acryloyloxydecyloxy]phenylcarbonyloxy)naphthalin;
1,5-bis(4-[11-Acryloyloxyundecyloxy]phenylcarbonyloxy)naphthalin;
1,5-bis(4-[12-Acryloyloxydodecyloxy]phenylcarbonyloxy)naphthalin.

### Beispiel 2

Zu einer Lösung von 1,25 g 4-[8-Vinyloxyoctyloxy)]benzoesäure und 0,25 g 1,4-Naphthohydrochinon , 0,2 g 4-Dimethylaminopyridin in 20 ml Dichlormethan wird unter Rühren bei Raumtemperatur 0,8 g N, N'-Dicyclohexyldicarbodiimid gegeben. Das Reaktionsgemisch wird über Nacht bei Raumtemperatur gerührt, auf 100 ml Wasser gegossen und dann dreimal mit je 50 ml Dichlormethan extrahiert. Die organischen Phasen werden vereinigt, zweimal mit je 100 ml Wasser gewaschen, über Magnesiumsulfat getrocknet , nitriert und das Filtrat anschliessend eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus Ethylalkohol ergibt 0,5 g 1,4-bis(4-[8-Vinyloxyoctyloxy]phenylcarbonyloxy)naphthalin.

In analoger Weise können folgende Verbindungen hergestellt werden:
1,4-bis(4-[3-Vinyloxypropyloxy]phenylcarbonyloxy)naphthalin;
1,4-bis(4-[4-Vinyloxybutyloxy]phenylcarbonyloxy)naphthalin;
1,4-bis(4-[5-Vinyloxypentyloxy]phenylcarbonyloxy)naphthalin;
1,4-bis(4-[6-Vinyloxyhexyloxy]phenylcarbonyloxy)naphthalin;
1,4-bis(4-[7-Vinyloxyheptyloxy]phenylcarbonyloxy)naphthalin;
1,4-bis(4-[9-Vinyloxynonyloxy]phenylcarbonyloxy)naphthalin;
1,4-bis(4-[10-Vinyloxydecyloxy]phenylcarbonyloxy)naphthalin;
1,4-bis(4-[11-Vinyloxyundecyloxy]phenylcarbonyloxy)naphthalin;
1,4-bis(4-[12-Vinyloxydodecyloxy]phenylcarbonyloxy)naphthalin;
1,5-bis(4-[3-Vinyloxypropyloxy]phenylcarbonyloxy)naphthalin;
1,5-bis(4-[4-Vinyloxybutyloxy]phenylcarbonyloxy)naphthalin;
1,5-bis(4-[5-Vinyloxypentyloxy]phenylcarbonyloxy)naphthalin;
1,5-bis(4-[6-Vinyloxyhexyloxy]phenylcarbonyloxy)naphthalin;
1,5-bis(4-[7-Vinyloxyheptyloxy]phenylcarbonyloxy)naphthalin;
1,5-bis(4-[8-Vinyloxyoctyloxy]phenylcarbonyloxy)naphthalin;
1,5-bis(4-[9-Vinyloxynonyloxy]phenylcarbonyloxy)naphthalin;
1,5-bis(4-[10-Vinyloxydecyloxy]phenylcarbonyloxy)naphthalin;
1,5-bis(4-[11-Vinyloxyundecyloxy]phenylcarbonyloxy)naphthalin;
1,5-bis(4-[12-Vinyloxydodecyloxy]phenylcarbonyloxy)naphthalin.

### Beispiel 3

Zu einer Lösung von 1,25 g 4-[7, 8-Oxiranoctyloxy)]benzoesäure und 0,25 g 1,4-Naphthohydrochinon, 0,2 g 4-Dimethylaminopyridin in 20 ml Dichlormethan wird unter Rühren bei Raumtemperatur 0,8 g N, N'-Dicyclohexyldicarbodiimid gegeben. Das Reaktionsgemisch wird über Nacht bei Raumtemperatur gerührt, auf 100 ml Wasser gegossen und dann dreimal mit je 50 ml Dichlormethan extrahiert. Die vereinigten organischen Phasen werden zweimal mit je 100 ml Wasser gewaschen, über Magnesiumsulfat getrocknet , filtriert und das Filtrat anschliessend eingeengt. Chromatographische Reinigung des Rückstandes an Kieselgel mit Cyclohexan/ Ethylacetat (Vol. 8:2) und zweimaliges Umkristallisieren der gemäss Dünnschichtchromatographie reinen Fraktionen aus Ethylalkohol ergibt 0,5 g 1,4-bis(4-[7,8-Oxiranoctyloxy]phenylcarbonyloxy)-naphthalin.

In analoger Weise können folgende Verbindungen hergestellt werden:
1,4-bis(4-[2, 3-Oxiranpropyloxy]phenylcarbonyloxy)naphthalin;
1,4-bis(4-[3, 4-Oxiranbutyloxy]phenylcarbonyloxy)naphthalin;
1,4-bis(4-[4, 5-Oxiranpentyloxy]phenylcarbonyloxy)naphthalin;
1,4-bis(4-[5, 6-Oxiranhexyloxy]phenylcarbonyloxy)naphthalin;
1,4-bis(4-[6, 7-Oxiranheptyloxy]phenylcarbonyloxy)naphthalin;
1,4-bis(4-[8, 9-Oxirannonyloxy]phenylcarbonyloxy)naphthalin;
1,4-bis(4-[9, 10-Oxirandecyloxy]phenylcarbonyloxy)naphthalin;
1,4-bis(4-[10, 11-Oxiranundecyloxy]phenylcarbonyloxy)naphthalin;
1,4-bis(4-[11,12-Oxirandodecyloxy]phenylcarbonyloxy)naphthalin;
1,5-bis(4-[2, 3-Oxiranpropyloxy]phenylcarbonyloxy)naphthalin;
1,5-bis(4-[3, 4-Oxiranbutyloxy]phenylcarbonyloxy)naphthalin;
1,5-bis(4-[4, 5-Oxiranpentyloxy]phenylcarbonyloxy)naphthalin;
1,5-bis(4-[5, 6-Oxiranhexyloxy]phenylcarbonyloxy) naphthalin;
1,5-bis(4-[6,7-Oxiranheptyloxy]phenylcarbonyloxy)naphthalin;
1,5-bis(4-[7, 8-Oxiranoctyloxy]phenylcarbonyloxy)naphthalin;
1,5-bis(4-[8, 9-Oxirannonyloxy]phenylcarbonyloxy)naphthalin;
1,5-bis(4-[9, 10-Oxirandecyloxy]phenylcarbonyloxy)naphthalin;
1,5-bis(4-[10, 11-Oxiranundecyloxy]phenylcarbonyloxy)naphthalin;
1,5-bis(4-[11,12-Oxirandodecyloxy]phenylcarbonyloxy)naphthalin.

### Beispiel 4

Ein Gemisch (C-N, <20°C, N-I, 105°C) aus 80 Gew.% 1,4-bis(4-[8-Acryloyloxyoctyloxy]phenylcarbonyloxy)naphthalin und 20 Gew.% 1-Chlor-2,5-bis(4-[6-acryloyloxyhexyloxy)]phenylcarbonyloxy)benzol wurde vorgelegt, mit 1 Gew.% eines Photoinitiators (IRGACURE, Ciba Geigy) versetzt, in Anisol gelöst (40 Gew.%) und dann bei 2000 Umdrehungen pro Minute auf eine Glasplatte geschleudert. Die Glasplatte wurde zuvor mit Poly-[Methacryloyloxyethyl-3-(E)-[4-cyano-4'-biphenyl]acrylat] beschichtet, wie in der Schweizerischen Patentanmeldung CH 2016/94 beschrieben, und dann mit linear polarisiertem Licht bestrahlt. Dabei wurde eine vorgegebene Struktur mittels einer Maske in die (PPN) Schicht photolithographisch eingeschrieben. Die neue Schicht (auf der PPN Schicht) wurde bei 90°C auf einer Wärmebank getrocknet, dann im Vakuumschrank unter Vakuum bei 90°C mit Xenon-licht belichtet. Die eingeschriebene Originalstruktur blieb erhalten und wurde vom neuen Netzwerk getreu übernommen. Eine klare Doppelbrechung (Δn) war erkennbar. Diese Schicht kann als ein strukturierter optischer Retarder verwendet werden.

## Patentansprüche

1. Verbindungen der allgemeinen Formel 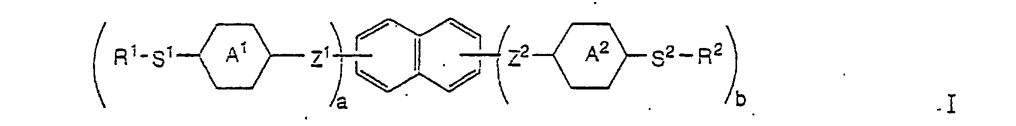 worin
R¹ und R² unabhängig voneinander eine vernetzbare Gruppe wie Acrylat, Methacrylat, 2-Chloracrylat, 2-Phenylacrylat, Epoxy oder Vinyloxy
S¹ und S² -(CY₂)ₘ-, -O(CY₂)ₘ-, -(CY₂)ₘO-, -(CY₂)ₘCOO-,
-(CY₂)ₘOOC-, -(Si[(CH₃)₂]O)ₘ-,
-OCH₂(Si[(CH₃)₂]O)ₘSi[(CH₃)₂]CH₂O-, oder
-NHCH₂(Si[(CH₃)₂]O)ₘSi[(CH₃)₂]CH₂NH-;
Y Wasserstoff, Fluor oder Methyl;
a, b 1;
m eine ganze Zahl von 1 bis 16;
A¹ und A² unabhängig voneinander gegebenenfalls mit Halogen, Cyano, Methyl; Methoxy und/oder Acetyl einfach oder mehrfach substituiertes 1,4-Phenylen, Pyridin-2, 5-diyl, Pyrimidin-2, 5-diyl, trans-1,4-Cyclohexylen oder trans-1,3-Dioxan-2, 5-diyl; und
Z¹ und Z² unabhängig voneinander eine Einfachbindung, -CH₂CH₂-, -OCH₂-, -CH₂O-, -COO-, -OOC-, -(CH₂)₄-, -O(CH₂)₃- oder -(CH₂)₃O- bedeuten, wobei der Naphthylring in Stellung 1 und 5, bzw. 1 und 4 mit Z¹ und Z² verknüpft ist.

2. Verbindungen nach Anspruch 1 , **dadurch gekennzeichnet, dass** die Reste R¹ und R² die gleiche Bedeutung haben.

3. Verbindungen nach einem der Ansprüche 1 oder 2 **dadurch gekennzeichnet, dass** S¹ und S² die gleiche Bedeutung haben.

4. Verbindungen nach einem der Ansprüche 1 bis 3, **dadurch gekennzeichnet, dass** Z¹ und Z² die gleiche Bedeutung haben und -OCH₂-, -CH₂O-, -COO- oder -OOC- bedeutet.

5. Verbindungen nach einem der Ansprüche 1 bis 4, **dadurch gekennzeichnet, dass** die Ringe A¹ und A² die gleiche Bedeutung haben und 1,4-Phenylen oder 2- bzw. 3-Fluor-1,4-phenylen bedeuten.

6. Verbindungen nach Anspruch 1 der allgemeinen Formeln 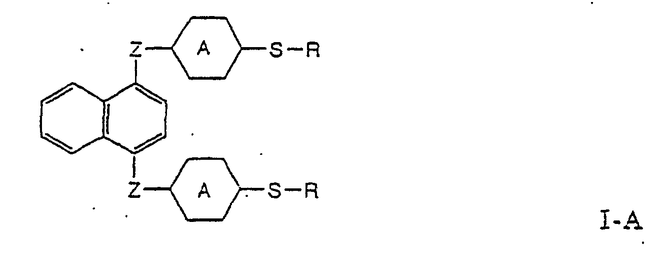 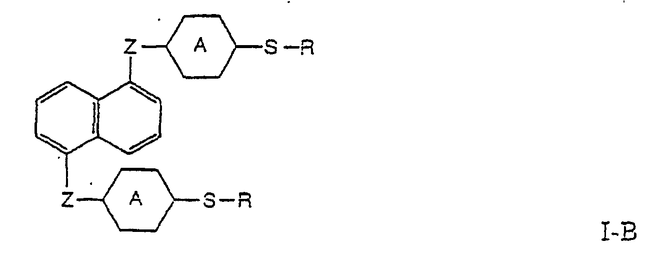 worin
R Acrylat, Methacrylat, 2-Chloracrylat, 2-Phenylacrylat, Vinyloxy oder Epoxy;
S -(CH₂)_{m'}-, -O(CH₂)_{m'}- oder -(CH₂)_{m'}O-;
m' eine ganze Zahl von 4 bis 12;
A 1,4-Phenylen oder 2- bzw. 3-Fluor-1,4-phenylen; und
Z -OCH₂- oder -OOC- bedeuten.

7. Vernetzbare Gemische bestehend aus mindestens 2 Komponenten, wovon mindestens eine Komponente eine Verbindung der in Anspruch 1 definierten Formel I ist.

8. Vernetzbare Gemische gemäss Anspruch 7, **dadurch gekennzeichnet, dass** sie neben einer oder mehreren Verbindungen der Formel I, eine oder mehrere Verbindungen aus der Gruppe der Formeln 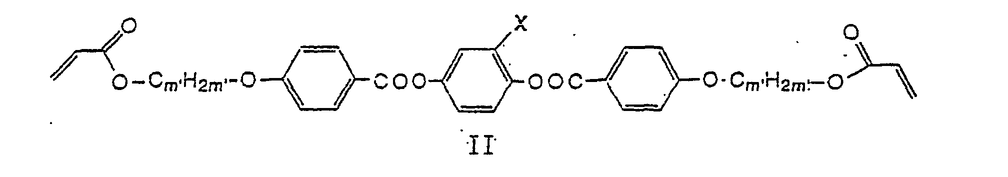 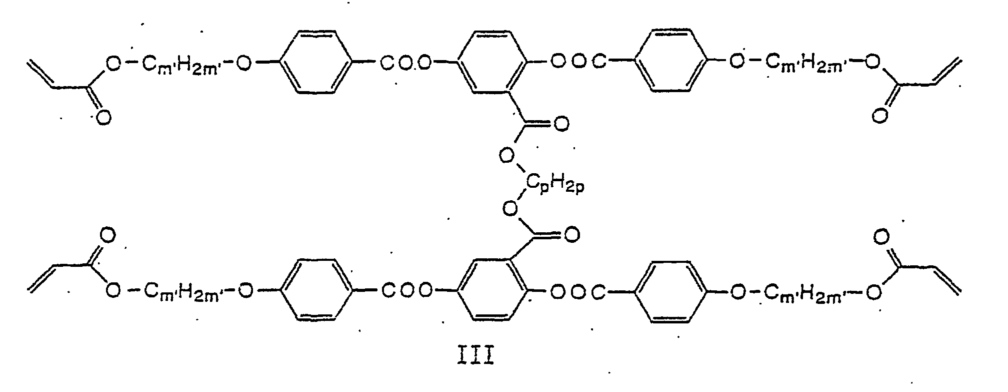 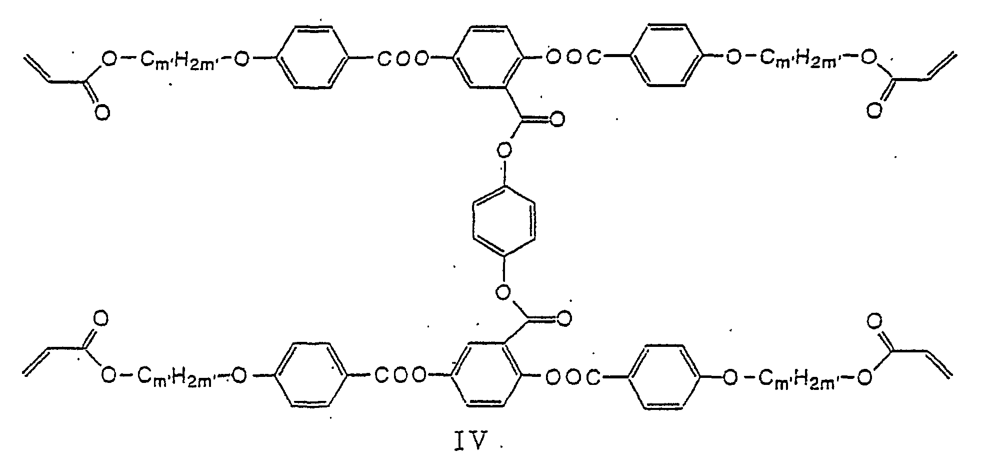 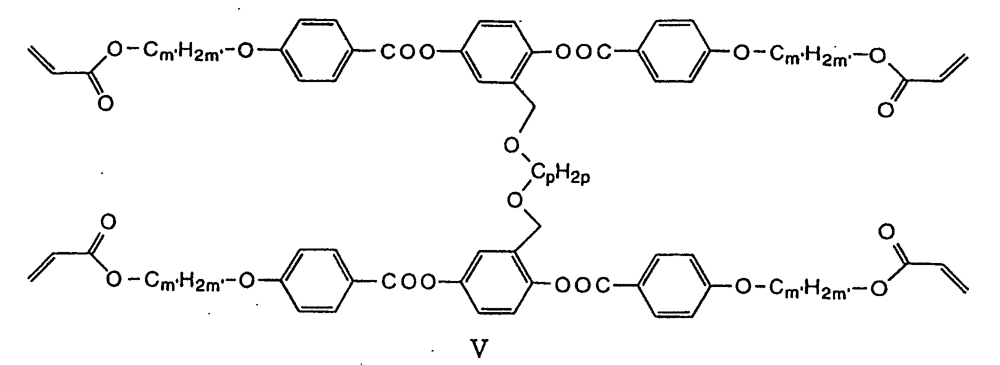 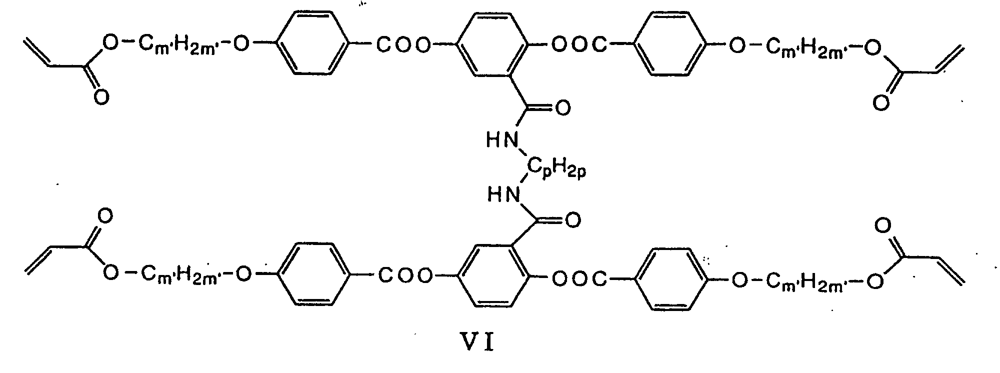 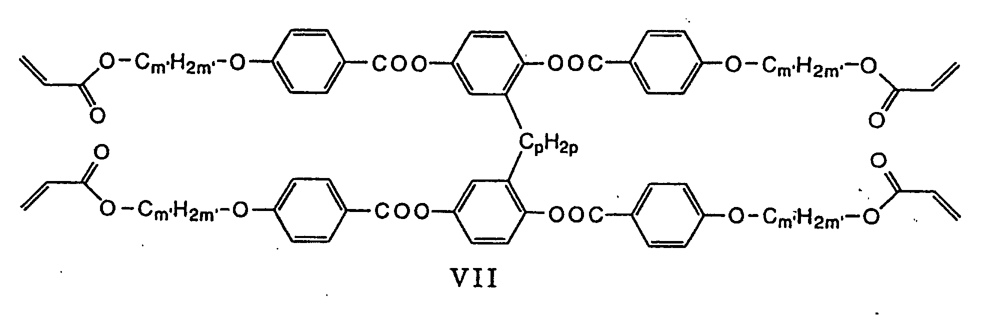 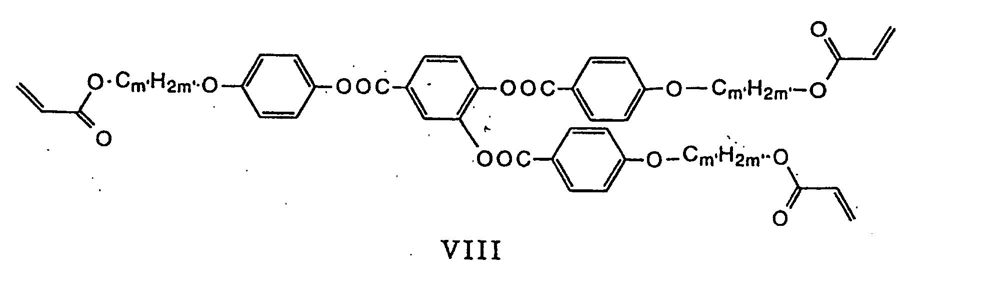 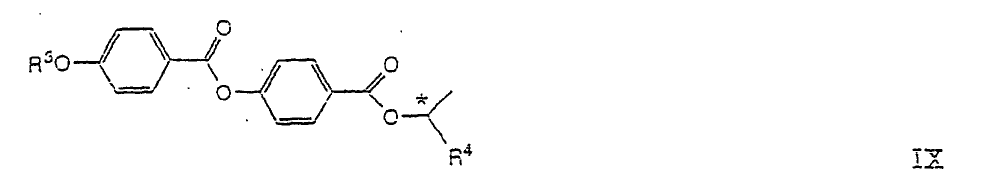 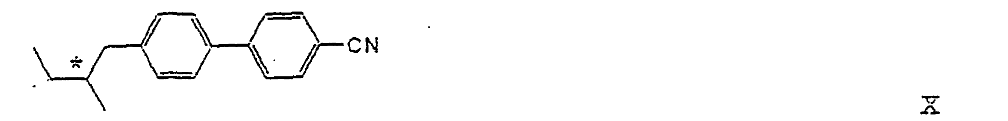 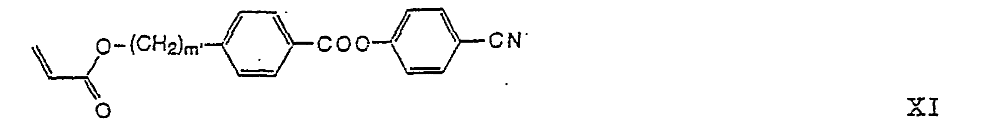 und 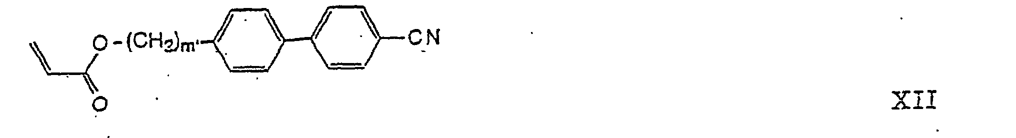 worin
p eine ganze Zahl von 2 bis 12;
R³ und R⁴ unabhängig voneinander Alkyl oder Alkenyl mit 2 bis 12 Kohlenstoffatomen;
X Wasserstoff, Methyl, Ethyl, Propyl, i-Propyl, Butyl, i-Butyl, tert.-Butyl, Fluor, Brom, Chlor oder Cyano;
m' eine ganze Zahl von 4 bis 12; und
* ein Chiralitätszentrum bedeuten.

9. Verwendung von Verbindungen gemäss einem der Ansprüche 1 bis 6 in ihrem vernetzten Zustand für optische Bauelemente.

10. Verwendung von vernetzbaren flüssigkristallinen Gemischen gemäss einem der Ansprüche 7 oder 8 in ihrem vernetzten Zustand für optische Bauelemente.

## Claims

1. A compound of the formula: 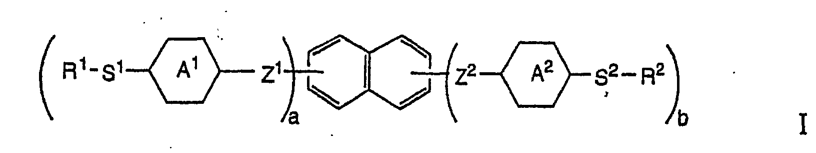 wherein
R¹ and R² each independently is a cross-linkable group such as acrylate, methacrylate, 2-chloroacrylate, 2-phenylacrylate, epoxy or vinyloxy
S¹ and S² each independently is -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, or -NHCH₂(Si[(CH₃)₂]O)ₘSi[(CH₃)₂]CH₂NH-;
Y is hydrogen, fluorine, or methyl;
a, b is 1;
m is a whole number of from 1 to 16;
A¹ and A² each independently is pyridine-2,5-diyl, pyrimidine-2,5-diyl, trans-1,4-cyclohexylene, trans-1,3-dioxane-2,5-diyl, or 1,4-phenylene which is unsubstituted, mono-substituted or multiply-substituted with one or more substituents selected from the group consisting of halogen, cyano, methyl, methoxy, and/or acetyl; and
Z¹ and Z² each independently is a single bond, -CH₂CH₂ -, -OCH₂-, -CH₂O-, -COO-, -OOC-, -(CH₂)₄-, -O(CH₂)₃-, or -(CH₂)₃O-, wherein the naphthyl ring is linked in position 1 and 5 or in position 1 and 4 with Z¹ and Z², respectively.

2. The compound according to claim 1, wherein R¹ and R² are the same.

3. The compound according to claims 1 or 2, wherein S¹ and S² are the same.

4. The compound according to anyone of claims 1 to 3, wherein Z¹ and Z² are the same and are selected from the group consisting of -OCH₂-, -CH₂O-, -COO- and -OOC-.

5. The compound according to anyone of claims 1 to 4, wherein rings A¹ and A² are the same and are selected from the group consisting of 1,4-phenylene, 2-fluoro-1,4-phenylene, and 3-fluoro-1,4-phenylene.

6. The compound according to claim 1 of the formulae: 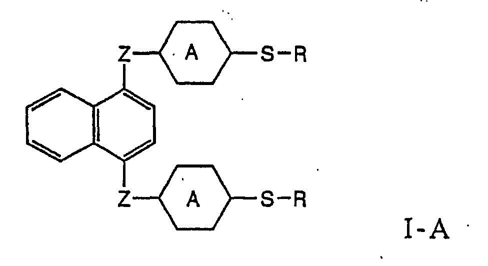 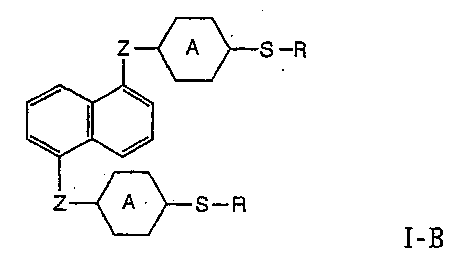 wherein
R is acrylate, methacrylate, 2-chloroacrylate, 2-phenylacrylate, vinyloxy, or epoxy;
S is -(CH₂)_{m'}-, -O(CH₂)_{m'}-, or -(CH₂)_{m'}O-;
m' is a whole number of from 4 to 12;
A is 1,4-phenylene, 2-fluoro-1,4-phenylene, or 3-fluoro-1,4-phenylene; and
Z is -OCH₂- or -OOC-.

7. A cross-linkable mixture consisting of at least 2 components, of which at least one component is a compound of formula I as claimed in claim 1.

8. The cross-linkable mixture according to claim 7 further comprising in addition to one or more compounds of formula I one or more compounds selected from the group consisting of: 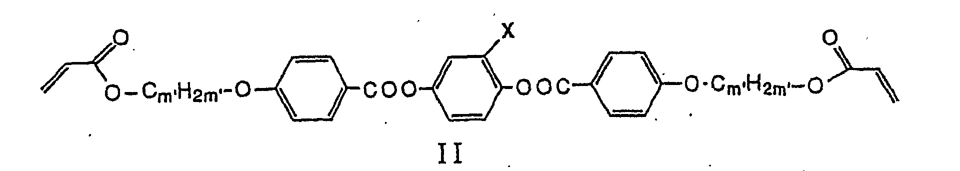 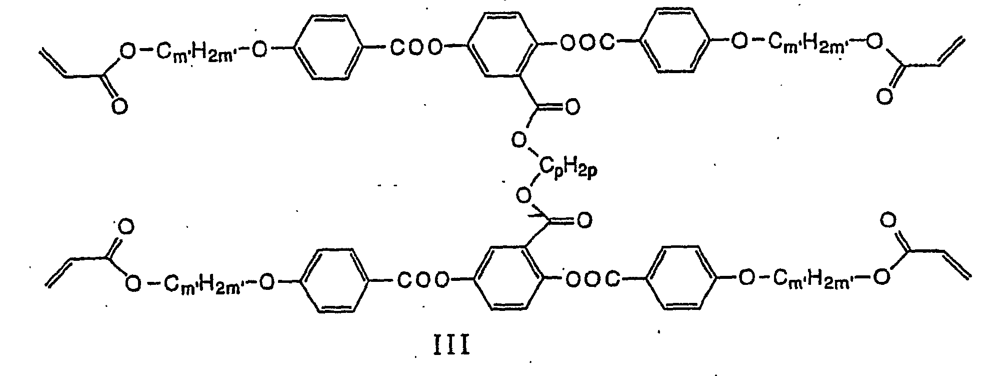 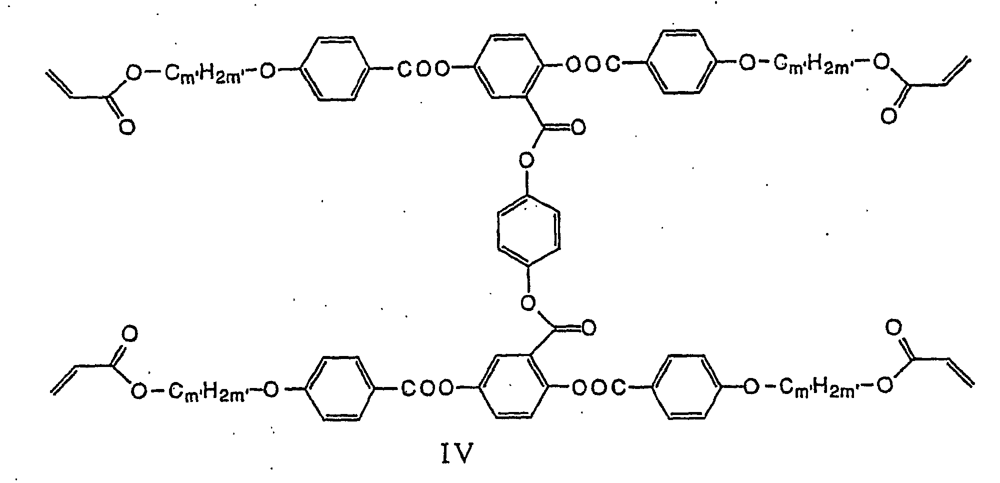 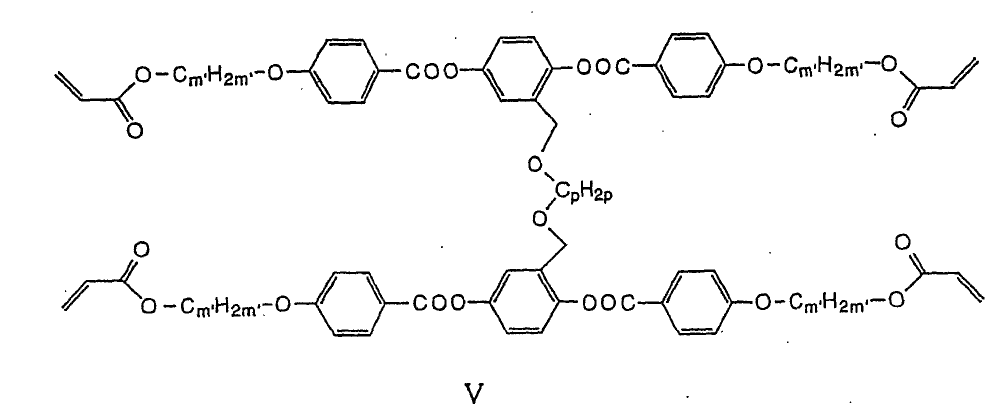 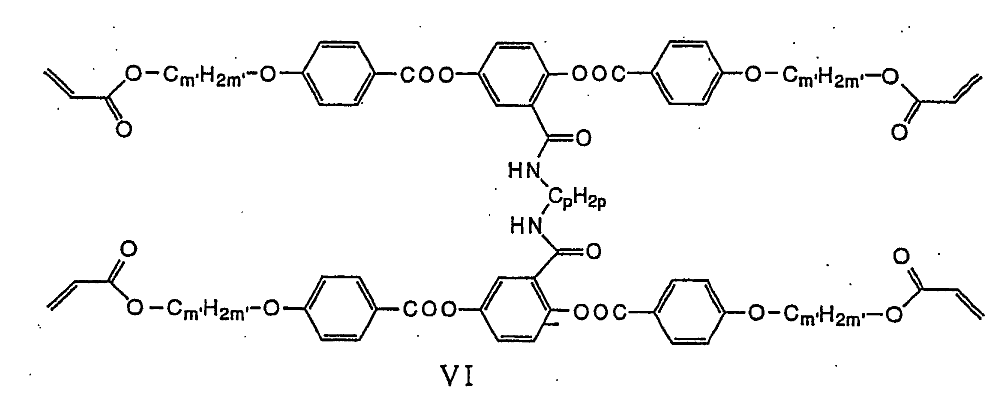 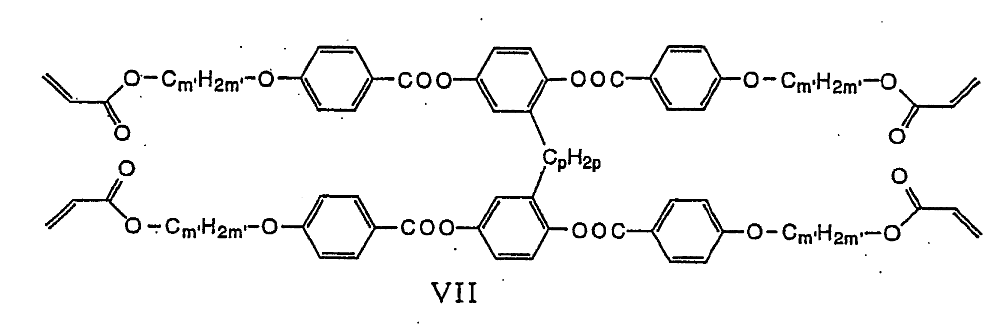 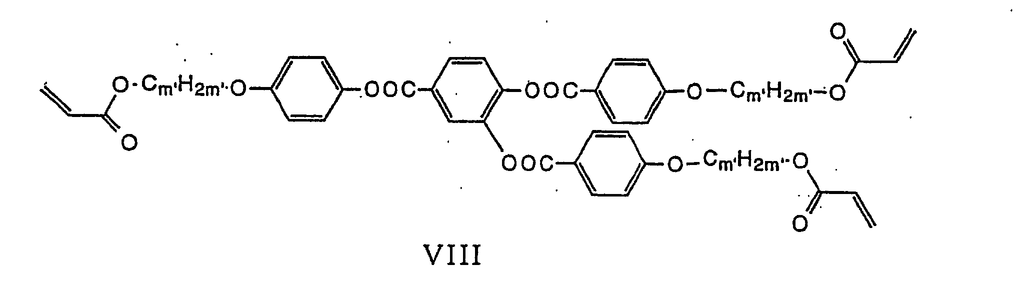 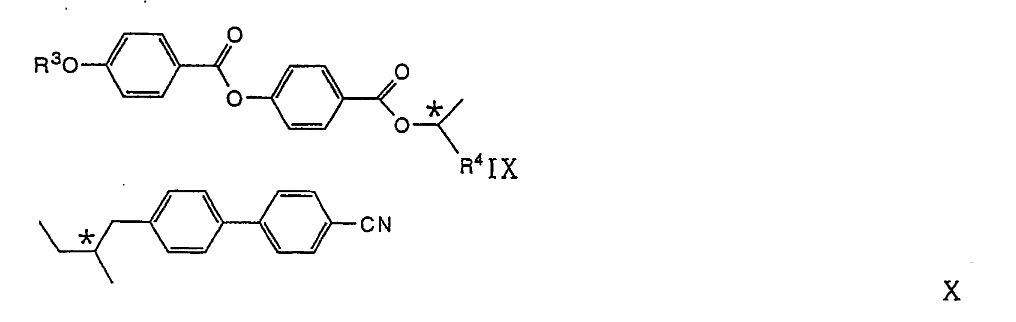 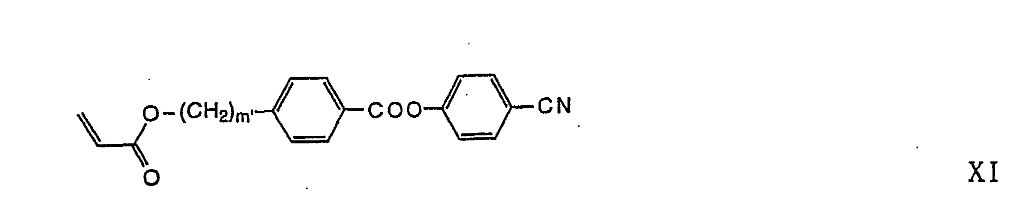 and 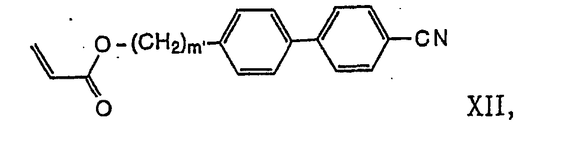 wherein
p signifies a whole number of 2 to 12;
R³ and R⁴ each independently signify alkyl or alkenyl with 2 to 12 carbon atoms;
X signifies hydrogen, methyl, ethyl, propyl, i-propyl, butyl, i-butyl, tert.-butyl, fluorine, bromine, chlorine or cyano;
m' signifies a whole number of 4 to 12; and
* signifies a center of chirality.

9. Use of compounds according to anyone of claims 1 to 6 in their crosslinked state for optical components.

10. Use of crosslinkable, liquid crystalline mixtures according to claims 7 or 8 in their crosslinked state for optical components.

## Revendications

1. Composés de formule générale 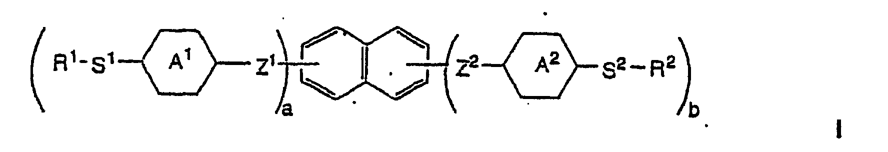 dans laquelle
R¹ et R² représentent indépendamment l'un de l'autre un groupe réticulable comme acrylate, méthacrylate, 2-chloroacrylate, 2-phénylacrylate, époxy ou vinyloxy
S¹ et S² représentent -(CY₂)ₘ-, -O(CY₂)ₘ-, -(CY₂)ₘO-, -(CY₂)ₘCOO-, -(CY₂)ₘOOC-, -Si[(CH₃)₂]O)ₘ-, -OCH₂(Si[(CH₃)₂]O)ₘSi[(CH₃)₂]CH₂O- ou -NHCH₂(Si[(CH₃)₂]OₘSi[(CH₃)₂)CH₂NH-;
Y représente un hydrogène, fluor ou méthyle ;
a, b représentent 1 ;
m représente un nombre entier de 1 à 16 ;
A¹ et A² représentent, indépendamment l'un de l'autre, un 1,4-phénylène éventuellement substitués une fois ou plusieurs fois par halogène, cyano, méthyle, méthoxy et/ou acétyle, pyridine-2,5-diyle, pyrimidine-2,5-diyle, trans-1,4-cyclohexylène ou trans-1,3-dioxane-2,5-diyle; et
Z¹ et Z² représentent, indépendamment l'un de l'autre, une liaison simple, -CH₂CH₂-, -OCH₂-, -CH₂O-, -COO-, -OOC-, -(CH₂)₄-, -O(CH₂)₃- ou -(CH₂)₃O-,
dans laquelle le cycle naphtyle est couplé en position 1 et 5, ou 1 et 4 avec Z¹ et Z².

2. Composés selon la revendication 1, **caractérisés en ce que** les groupes R¹ et R² ont la même signification.

3. Composés selon une des revendications 1 ou 2, **caractérisés en ce que** S¹ et S² ont la même signification.

4. Composés selon une des revendications 1 à 3, **caractérisés en ce que** Z¹ et Z² ont la même signification et représentent -OCH₂-, -CH₂O-, -COO- ou -OOC-.

5. Composés selon une des revendications 1 à 4, **caractérisés en ce que** les cycles A¹ et A² ont la même signification et représentent le 1,4-phénylène ou le 2- ou 3-fluoro-1,4-phénylène.

6. Composés selon la revendication 1 de formules générales 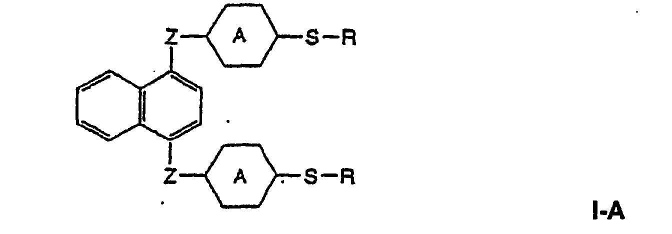 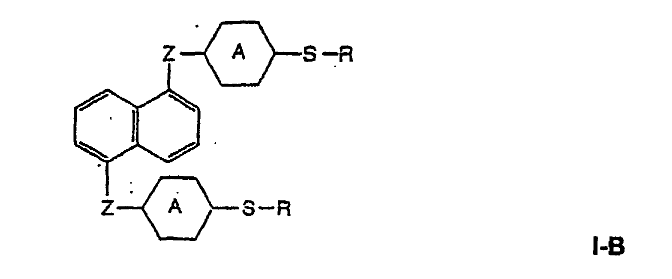 dans lesquelles
R représente un acrylate, méthacrylate, 2-chloroacrylate, 2-phénylacrylate, vinyloxy ou époxy ;
S représente -(CH₂)_{m'}-, -O(CH₂)_{m'}- ou -(CH₂)_{m'}O- ;
m' représente un nombre entier de 4 à 12 ;
A représente un 1,4-phénylène ou 2- ou 3-fluoro-1,4-phénylène ; et
Z représente -OCH₂- ou -OOC-.

7. Mélanges réticulables constitués d'au moins 2 composants, parmi lesquels au moins un composant est un composé de la formule I définie à la revendication 1.

8. Mélanges réticulables selon la revendication 7, **caractérisés en ce qu'**ils contiennent en plus d'un ou de plusieurs composés de formule I, un ou plusieurs composés choisis dans le groupe des formules 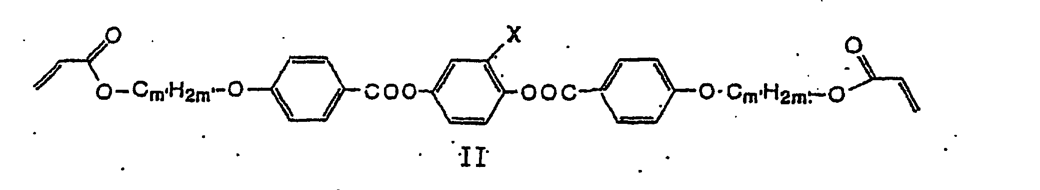 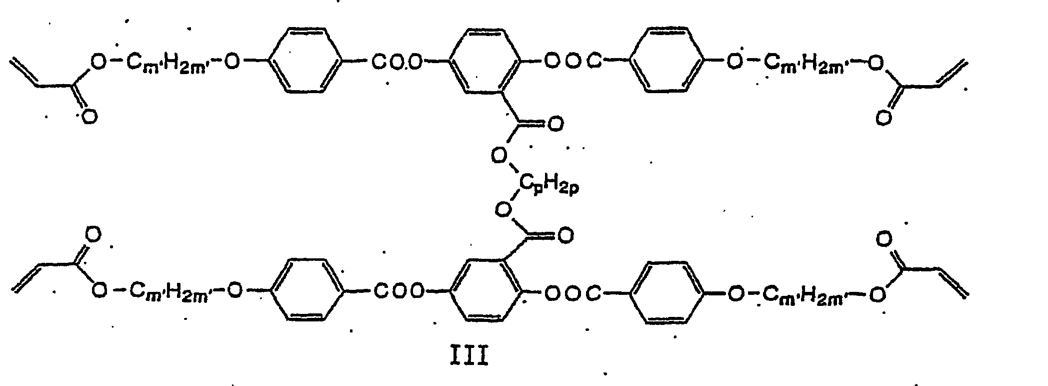 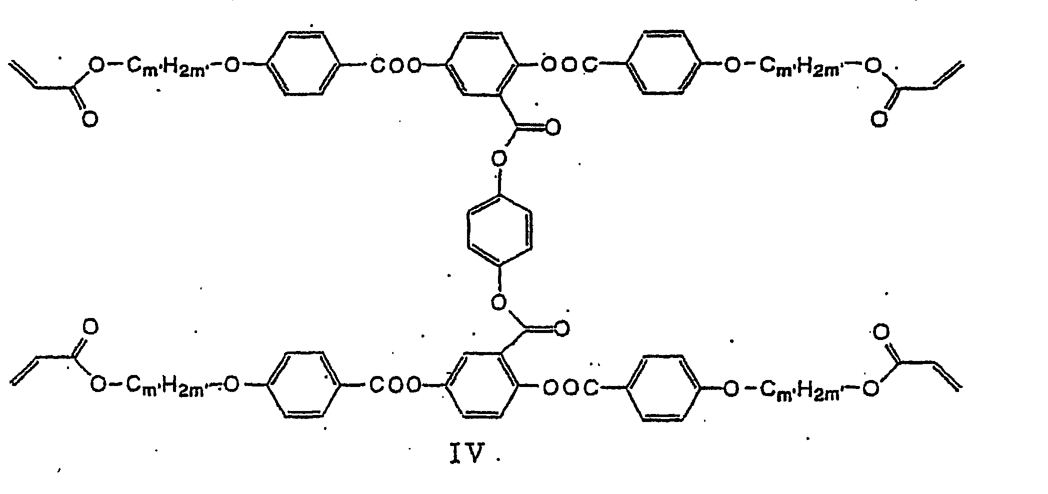 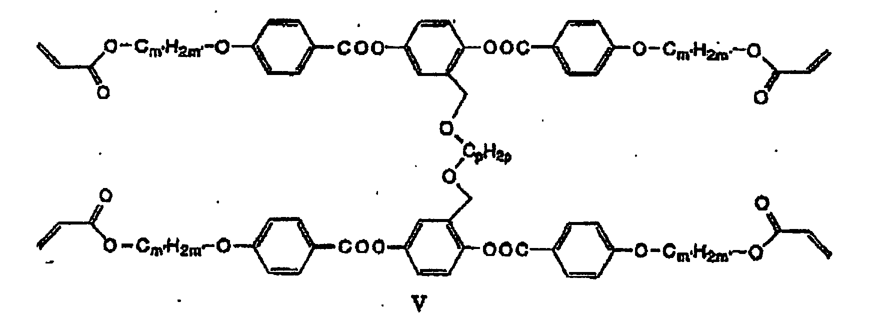 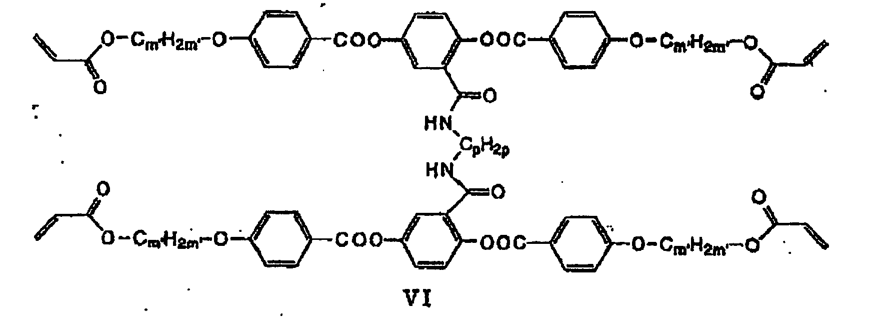 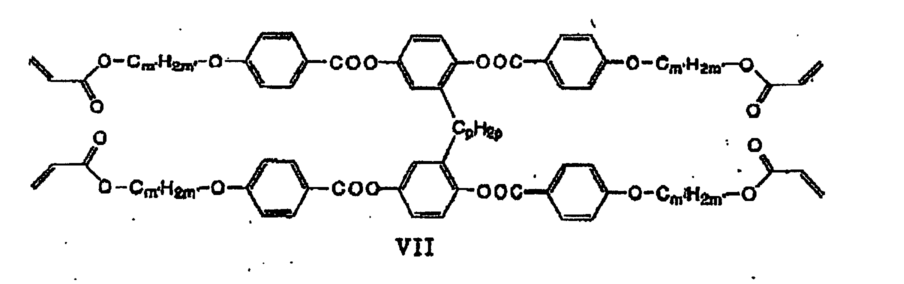 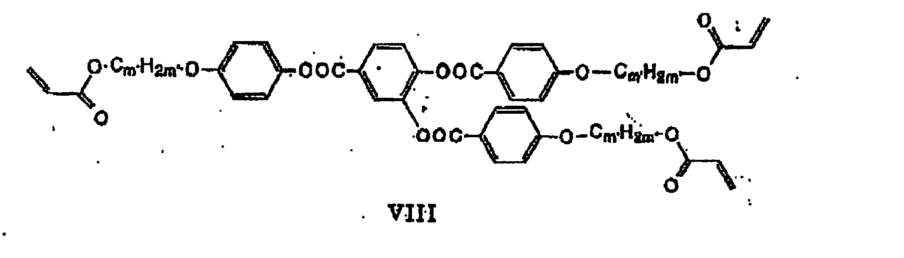 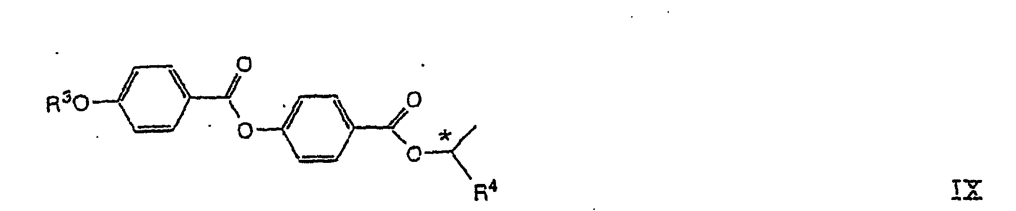 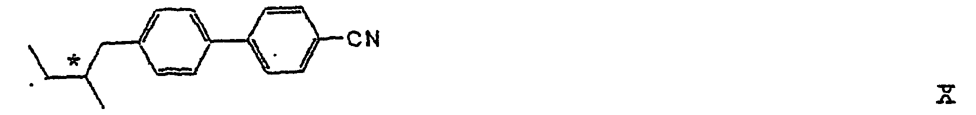 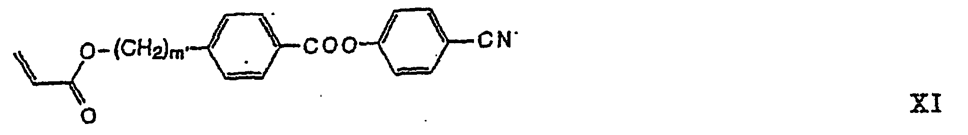 et 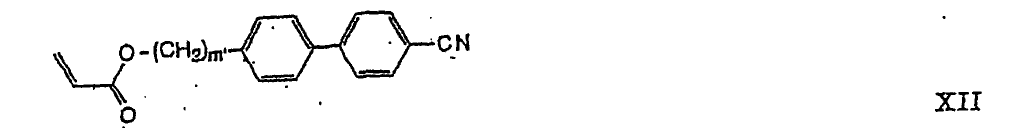 dans lesquelles
p représente un nombre entier de 2 à 12 ;
R³ et R⁴ représentent indépendamment l'un de l'autre un alkyle ou alcényle ayant de 2 à 12 atomes de carbone ;
X représente un hydrogène, méthyle, éthyle, propyle, i-propyle, butyle, i-butyle, tert.-butyle, fluor, brome, chlore ou cyano ;
m' représente un nombre entier de 4 à 12 ; et
* représente un centre de chiralité.

9. Utilisation de composés selon l'une des revendications 1 à 6 dans leur état réticulé pour des composants optiques.

10. Utilisation de mélanges de cristaux liquides réticulables selon une des revendications 7 ou 8 dans leur état réticulé pour des composants optiques.
